(19) Europäisches Patentamt / European Patent Office / Office européen des brevets

(11) **EP 2 415 439 B1**

(12) **EUROPEAN PATENT SPECIFICATION**

(45) Date of publication and mention of the grant of the patent:
**19.11.2014 Bulletin 2014/47**

(21) Application number: **10758527.5**

(22) Date of filing: **25.03.2010**

(51) Int Cl.:
*A61F 13/49* (2006.01)      *A61F 13/53* (2006.01)

(86) International application number:
**PCT/JP2010/055221**

(87) International publication number:
**WO 2010/113754 (07.10.2010 Gazette 2010/40)**

(54) **WATER-ABSORBENT SHEET**

WASSERABSORBIERENDE FOLIE

FEUILLET ABSORBANT L'EAU

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **31.03.2009 JP 2009085432**

(43) Date of publication of application:
**08.02.2012 Bulletin 2012/06**

(73) Proprietor: **Sumitomo Seika Chemicals Co., Ltd. Hyogo 675-0145 (JP)**

(72) Inventors:
  • **TAKATORI, Junichi**
    **Himeji-shi**
    **Hyogo 672-8076 (JP)**
  • **SAKATA, Jun**
    **Himeji-shi**
    **Hyogo 672-8076 (JP)**
  • **INABA, Haruka**
    **Himeji-shi**
    **Hyogo 672-8076 (JP)**
  • **MATSUSHITA, Hideki**
    **Himeji-shi**
    **Hyogo 672-8076 (JP)**

(74) Representative: **Hoffmann Eitle Patent- und Rechtsanwälte PartmbB Arabellastraße 30 81925 München (DE)**

(56) References cited:
**EP-A2- 0 829 245          WO-A1-2007/049725**
**JP-A- 2007 117 117       US-A- 3 881 490**
**US-A1- 2004 116 884**

**Description**

TECHNICAL FIELD

The present invention relates to a thin water-absorbent sheet which can be used in the fields of hygienic materials and the like. More specifically, the present invention relates to a water-absorbent sheet containing a very small amount of pulp, which can be suitably used in absorbent articles, such as disposable diapers and incontinence pads, which has a fast permeation rate of a liquid and does not cause liquid leakage. Furthermore, the present invention relates to an absorbent article obtainable from the water-absorbent sheet.

BACKGROUND ART

Body liquid absorbent articles usable in disposable diapers or the like comprise an absorbent material for absorbing a liquid such as a body liquid, a flexible liquid-permeable surface sheet (top sheet) positioned on a side contacting a body, and a liquid-impermeable backside sheet (back sheet) positioned on a side opposite to that contacting the body.

Conventionally, there have been increasing demands for thinning and light-weighing of absorbent articles, from the viewpoint of designing property and c+onvenience upon carrying, and efficiency upon distribution. Further, in the recent years, there have been growing needs for so-called eco-friendly intentions, in which resources are effectively utilized so that use of natural materials that require a long time to grow such as trees is avoided as much as possible, from the viewpoint of environmental protection. Conventionally, a method for thinning that is generally carried out in absorbent articles is a method of reducing hydrophilic fibers such as crushed pulp of a wood material, which has a role of fixing a water-absorbent resin in an absorbent material, while increasing a water-absorbent resin.

An absorbent material in which a water-absorbent resin is used in a large amount with a lowered proportion of a hydrophilic fiber is preferred in thinning, from the viewpoint of reducing bulky hydrophilic fibers while retaining a liquid. However, when distribution or diffusion of a liquid upon actually using in an absorbent article such as disposable diapers is considered, there is a disadvantage that if a large amount of the water-absorbent resin is formed into a soft gel-like state by absorption, a so-called "gel-blocking phenomenon" takes place, whereby liquid diffusibility is markedly lowered and a liquid permeation rate of the absorbent material is slowed down. This "gel-blocking phenomenon" is a phenomenon in which especially when an absorbent material in which water-absorbent resins are highly densified absorbs a liquid, a water-absorbent resin existing near a surface layer absorbs the liquid to even more densify soft gel that forms near the surface layer, so that a liquid permeation into an internal of an absorbent material is inhibited, thereby making the internal of the water-absorbent resin incapable of efficiently absorbing the liquid.

In view of the above, conventionally, as a means of inhibiting gel blocking phenomenon which takes place by reducing hydrophilic fibers while using a water-absorbent resin in a large amount, for example, proposals such as a method using an absorbent polymer having such properties as specified Saline Flow Conductivity and Performance under Pressure (see Patent Publication 1), and a method using a water-absorbent resin prepared by heat-treating a specified water-absorbent resin precursor with a specified surface crosslinking agent (see Patent Publication 2) have been made.

However, in these methods, the absorption properties as absorbent materials in which water-absorbent resins are used in large amounts are not satisfactory. In addition, there arise some problems that the water-absorbent resin is subjected to be mobile before use or during use because hydrophilic fibers that play a role of fixing the water-absorbent resin are reduced. The absorbent material in which the localization of the absorbent resin takes place is more likely to cause gel-blocking phenomenon.

Further, in an absorbent material of which hydrophilic fibers that contribute to retention of the form are reduced has a lowered strength as an absorbent material, deformation such as twist-bending or tear before or after the absorption of a liquid is likely to take place. In an absorbent material with deformation, liquid diffusibility has markedly lowered, so that abilities inherently owned by the absorbent material cannot be exhibited. In order to try to avoid such phenomena, a ratio of hydrophilic fibers and a water-absorbent resin would be limited, thereby posing limitations in the thinning of an absorbent article.

Even in a case where gel blocking phenomenon does not take place, a thing that would serve the role of conventional hydrophilic fibers by which a body fluid such as urea is temporarily subjected to water retention and diffusion of the liquid to an overall absorbent material is lacking, so that a liquid leakage is likely to occur in the absorbent laminate, without being able to sufficiently capture the liquid.

Further, techniques of providing embossment to an absorbent material part in an absorbent article are disclosed, for the purpose of improving a permeation rate of a body fluid, preventing a liquid leakage of a body liquid, and preventing shape deformation of an absorbent material (see Patent Publication 3).

PRIOR ART PUBLICATIONS

PATENT PUBLICATIONS

Patent Publication 1: Japanese Unexamined Patent Publication No. Hei-9-510889
Patent Publication 2: Japanese Patent Laid-Open No. Hei-8-57311
Patent Publication 3: Japanese Patent Laid-Open No. Hei-10-272155

SUMMARY OF THE INVENTION

PROBLEMS TO BE SOLVED BY THE INVENTION

An object of the present invention is to provide a water-absorbent sheet which can be suitably used in absorbent articles such as disposable diapers, which has a fast permeation rate of a liquid such as urine, and is capable of preventing a liquid leakage from an absorbent material, even for a water-absorbent sheet containing a very small amount of pulps. A further object of the present invention is provide an absorbent article obtained from the water-absorbent sheet.

MEANS TO SOLVE THE PROBLEMS

Specifically, the gist of the present invention relates to:

[1] a water-absorbent sheet comprising a structure comprising at least an absorbent layer containing a water-absorbent resin, sandwiched between two or more sheets of a hydrophilic nonwoven fabric,
wherein a central region W1 along a longitudinal direction of a water-absorbent sheet **1** is subjected to:

one or more continuous wavy embossing **2** that is non-overlapping with each other, extending along the longitudinal direction, and linear embossing **4** in a direction from each of tips **3** of wavy forms formed by the wavy embossing **2** towards an edge portion contouring along the longitudinal direction of the water-absorbent sheet **1,** and

wherein a branched structure formed by the wavy embossing **2** and the linear embossing **4** has an approximate Y-shape-form, and wherein the edge portion contouring along the longitudinal direction of the water-absorbent sheet **1** contains non-embossing regions W2, W2' not provided with embossing extending along the longitudinal direction, and further
wherein an areal percentage of embossment provided on the water-absorbent sheet **1** is from 3 to 25% of an entire area of the water-absorbent sheet **1**; and
[2] an absorbent article comprising the water-absorbent sheet as defined in the above [1], sandwiched between a liquid-permeable sheet and a liquid-impermeable sheet.

EFFECTS OF THE INVENTION

According to the present invention, effects are exhibited that a water-absorbent sheet that has a fast permeation rate of a liquid but does not cause a liquid leakage can be provided by providing the water-absorbent sheet with a specified embossing shape, even for a water-absorbent sheet containing a very small amount of pulps. Therefore, an absorbent article having excellent absorption properties can be provided by incorporating the water-absorbent sheet into the absorbent article as an absorbent material for disposable diapers and the like.

BRIEF DESCRIPTION OF THE DRAWINGS

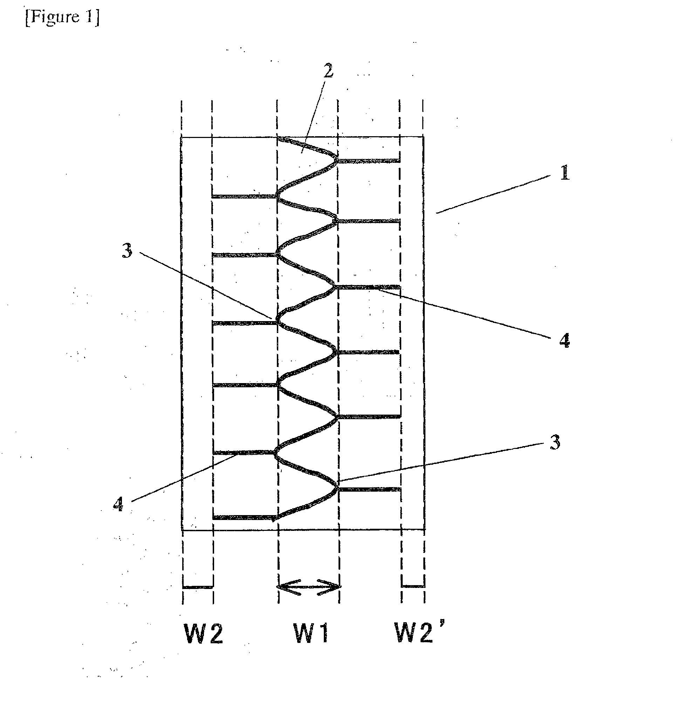

[Figure 1] The figure is a plan view schematically showing one example of a water-absorbent sheet of the present invention. In the water-absorbent sheet **1,** a central region W1 along a longitudinal direction of the water-absorbent sheet **1** is subjected to one continuous wavy embossing **2** extending along the longitudinal direction, and linear embossing **4** in a direction from each tips **3** of wavy forms formed by the wavy embossing **2** towards an edge portion contouring along the longitudinal direction of the water-absorbent sheet **1.** Further, the edge portion contouring along the longitudinal direction of the water-absorbent sheet **1** comprises non-embossing regions (W2, W2') not provided with embossing, extending along the longitudinal direction. Moreover, a branched structure of embossing formed by the wavy embossing **2** and the linear embossing **4** has an approximate Y-shaped form.

[Figure 2]The figure is a schematic view of an apparatus used for carrying out a slope leakage test.

[Figure 3] The figure is a plan view of a water-absorbent sheet showing one example (Example 1) of the present invention.

[Figure 4] The figure is a plan view of a water-absorbent sheet showing another example (Example 2) of the present invention.

[Figure 5] The figure is a plan view of a water-absorbent sheet showing another example (Example 3) of the present invention.

[Figure 6] The figure is a plan view of a water-absorbent sheet showing another example (Example 4) of the present invention.

[Figure 7] The figure is a plan view of a water-absorbent sheet showing another example (Example 5) of the present invention.

[Figure 8] The figure is a plan view of a water-absorbent sheet showing another example (Example 6) of the present invention.

[Figure 9] The figure is a plan view of a water-absorbent sheet showing another example (Example 7) of the present invention.

[Figure 10] The figure is a plan view of a water-absorbent sheet showing a comparative example (Comparative Example 2) of the present invention.

[Figure 11] The figure is a plan view of a water-absorbent sheet showing another comparative example (Comparative Example 3) of the present invention.

[Figure 12] The figure is a plan view of a water-absorbent sheet showing another comparative example (Comparative Example 4) of the present invention.

[Figure 13] The figure is a plan view of a water-absorbent sheet showing another comparative example (Comparative Example 5) of the present invention.

[Figure 14] The figure is a plan view of a water-absorbent sheet showing another comparative example (Comparative Example 6) of the present invention.

[Figure 15] The figure is a plan view of a water-absorbent sheet showing another comparative example (Comparative Example 7) of the present invention.

## MODES FOR CARRYING OUT THE INVENTION

The water-absorbent sheet of the present invention has a structure comprising an absorbent layer containing at least a water-absorbent resin, sandwiched with two or more sheets of a hydrophilic nonwoven fabric, and further provided with a specified embossing shape.

As the water-absorbent resins used in the present invention, known water-absorbent resins can be used. For example, the water absorbent resin includes hydrolysates of starch-acrylonitrile graft copolymers, neutralized products of starch-acrylic acid graft polymers, saponified products of vinyl acetate-acrylic acid ester copolymers, partially neutralized products of polyacrylic acid, and the like. Among them, the partially neutralized products of polyacrylic acids are preferred, from the industrial viewpoint such as supply capacity and costs. Methods for synthesizing partially neutralized products of polyacrylic acid include reversed phase suspension polymerization method and aqueous solution polymerization method. Among them, the water-absorbent resins obtained according to reversed phase suspension polymerization method are more preferably used, from the viewpoint of excellent flowability of the resulting particles, smaller amounts of fine powder, high water-absorbent properties, such as water absorption capacity and water-absorption rate.

The water-absorbent resin used in the present invention has a basis weight of preferably from 100 to 800 $g/m^2$, more preferably from 200 to 700 $g/m^2$, even more preferably 250 to 600 $g/m^2$, and more preferably from 270 to 550$g/m^2$, from the viewpoint of obtaining a sufficient water-absorbent capability as an absorbent material for disposable diapers and the like. The water-absorbent resin has a basis weight of preferably 100 $g/m^2$ or more, from the viewpoint of exhibiting sufficient water-absorbent capacities, and preferably 800 $g/m^2$ or less, from the viewpoint of inhibiting the occurrence of the "gel-blocking phenomenon" mentioned above, obtaining liquid diffusibility as an absorbent material, and suppressing the lowering of permeation rate.

The hydrophilic nonwoven fabric used in the present invention is not particularly limited, as long as the hydrophilic nonwoven fabric is a known nonwoven fabric in the field of art. The hydrophilic nonwoven fabric includes nonwoven fabrics made of polyolefin fibers such as polyethylene (PE) and polypropylene (PP); polyester fibers such as polyethylene terephthalate (PET), polytrimethylene terephthalate (PTT), and polyethylene naphthalate (PEN); polyamide fibers such as nylon; rayon fibers, and other synthetic fibers; nonwoven fabrics produced by mixing cotton, silk, hemp, pulp (cellulose) fibers, or the like, from the viewpoint of liquid permeability, flexibility and strength upon forming into a sheet, and the hydrophilic nonwoven fabric may be a mixture of two or more kinds of fibers. In addition, its surface may be subjected to a hydrophilic treatment according to a known method, as occasion demands. The nonwoven fabric made of synthetic fibers is preferably used, from the viewpoint of increasing the strength of the water-absorbent sheet, and especially at least one member selected from the group consisting of rayon fibers, polyolefin fibers, polyester fibers, and mixtures thereof is preferred. The hydrophilic nonwoven fabric made of synthetic fibers may contain pulp fibers in a small amount to an extent that the thickness of the water-absorbent sheet would not increase.

The hydrophilic nonwoven fabric in the present invention is preferably a nonwoven fabric having an appropriate basis weight and an appropriate thickness, from the viewpoint of giving the water-absorbent sheet of the present invention excellent liquid permeability, flexibility, strength and cushioning property, and from the viewpoint of speeding up the permeation rate of the water-absorbent sheet. The hydrophilic nonwoven fabric has a basis weight of preferably 15 g/m$^2$ or more, more preferably in the range of from 35 to 250 g/m$^2$, and even more preferably in the range of from 45 to 150 g/m$^2$. The hydrophilic nonwoven fabric has a thickness preferably in the range of from 200 to 1500 $\mu$m more preferably in the range of from 250 to 1200 $\mu$m, and even more preferably in the range of from 300 to 1000 $\mu$m.

The absorbent layer in the present invention comprises at least a water-absorbent resin, including, for example, one comprising one or more kinds of water-absorbent resins, one comprising a mixture of a water-absorbent resin with a small amount of a hydrophilic fiber or synthetic fiber in a level that would not increase thickness of a water-absorbent resin and a water-absorbent sheet, and one comprising a mixture of a water-absorbent resin and an adhesive. Among them, one comprising a mixture of a water-absorbent resin and an adhesive is preferred, from the viewpoint of immobilizing a water-absorbent resin, thereby preventing the water-absorbent resin to migrate before or during use within the water-absorbent sheet and causing localization thereof.

In addition, this absorbent layer may be a single layer or a plural layers fractionated with a hydrophilic nonwoven fabric.

In a case where an adhesive is used, the adhesive used includes, for example, rubber adhesives such as natural rubbers, butyl rubbers, and polyisoprene; styrenic elastomer adhesives such as styrene-isoprene block copolymers (SIS), styrene-butadiene block copolymers (SBS), styrene-isobutylene block copolymers (SIBS), and styrene-ethylene-butylene-styrene block copolymers (SEBS); ethylene-vinyl acetate copolymer (EVA) adhesives; ethylene-acrylic acid derivative copolymer adhesives such as ethylene-ethyl acrylate copolymer (EEA), and ethylene-butyl acrylate copolymer (EBA); ethylene-acrylic acid copolymer (EAA) adhesives; polyamide adhesives such as copolymer nylons and dimer acids-based polyamides; polyolefin adhesives such as polyethylenes, polypropylenes, atactic polypropylenes, and copolymeric polyolefins; polyester adhesives such as polyethylene terephthalate (PET), polybutylene terephthalate (PBT), and copolymeric polyesters; and acrylic adhesives, and these adhesives may be used together in two or more kinds. In the present invention, the ethylene-vinyl acetate copolymer adhesives, the styrenic elastomer adhesives, the polyolefinic adhesives, and the polyester adhesives are preferred, from the viewpoint of high adhesive strength, thereby making it possible to prevent exfoliation of a hydrophilic nonwoven fabric and scattering of the water-absorbent resin in the water-absorbent sheet.

The adhesive used has a melting temperature or a softening point of preferably from 60° to 180°C, and more preferably from 70° to 150°C, from the viewpoint of sufficiently fixing a water-absorbent resin to a nonwoven fabric, and at the same time preventing thermal deterioration or deformation of the nonwoven fabric. Here, in the water-absorbent sheet of the present invention, as the condition of the adhesive, the adhesive is in a state that the adhesive is adhered to a nonwoven fabric or a hydrophilic resin in a solid state by cooling the molten adhesive after melting in the process of producing a water-absorbent sheet.

The adhesive used is contained in an amount preferably in the range of from 0.05 to 2.0 times, more preferably in the range of from 0.08 to 1.5 times, and even more preferably in the range of from 0.1 to 1.0 time the amount of the water-absorbent resin contained (mass basis). It is preferable that the adhesive is contained in an amount of 0.05 times or more, from the viewpoint of having sufficient adhesion, thereby preventing exfoliation of the hydrophilic nonwoven fabrics themselves or scattering of the water-absorbent resin, and increasing strength of a water-absorbent sheet. It is preferable that the adhesive is contained in an amount of 2.0 times or less, from the viewpoint of avoiding the inhibition of the swelling of the water-absorbent resin caused by too strong adhesion to each other, thereby improving a permeation rate or liquid leakage of a water-absorbent sheet.

The water-absorbent sheet according to the present invention is provided with continuous wavy embossing in a central region along a longitudinal direction of the water-absorbent sheet, thereby allowing a liquid to diffuse in the longitudinal direction. This continuous embossing is considered to play a role of a pathway for allowing a large amount of liquids to flow (liquid transport pathway); in a case where a sheet is provided with embossing that is non-continuous, a liquid once stops at a part where embossing breaks apart, so that the liquid would spread to its surroundings, so that it takes time to reach the next embossing part, so that a permeation rate would also be delayed. In addition, in a case where the embossing provided in a central region along a longitudinal direction is not wavy but linear, the distance for a liquid transport pathway would be short, so that a liquid may diffuse before allowing it to be absorbed to a water-absorbent sheet, thereby causing a liquid leakage from a longitudinal direction of a water-absorbent sheet.

The continuous wavy embossing in the present invention may be at least one line, or may be plural lines that do not overlap with each other. It is preferable that the wavy embossing are plural lines because diffusion efficiency is improved by providing plural liquid transport pathways, so that a permeation rate would be faster. However, it is preferable that the number of lines is smaller, from the viewpoint of suppressing slope leakage caused by increase in the number of lines. In view of the above, the number of lines of the wavy embossing is preferably from 1 to 3 lines, and more preferably 1 line. In a case where embossing parts overlap with each other even partly, the surrounding of the overlapping parts would form a spatially closed region such as a sac. Inside this closed region, it would be in a state that the region is pressed down to upper and lower layers of a hydrophilic nonwoven fabric when a water-absorbent resin of an absorbent layer absorbs to be swollen, so that the swelling of a water-absorbent resin would be inhibited. As a result, inside this closed region, "gel blocking phenomenon" is likely to be caused, so that water absorption abilities of a water-absorbent sheet is lowered, such as a delayed permeation rate. Therefore, in the present invention, it is preferable that wavy (and linear) embossing is provided so as not to form the closed region as described above. The shape of the wavy embossing in the present specification, in other words the shape of embossing between each of the tips of the wavy forms formed by wavy embossing, is not particularly limited, and it may be linear or curved.

Next, embossing of a water-absorbent sheet of the present invention will be explained with reference to Figure 1.

A central region W1 of a water-absorbent sheet provided with continuous wavy embossing in the present invention, shown schematically in Figure 1, has a width of preferably within the range of from 0.15 to 0.45 times, and even more preferably from 0.2 to 0.4 times, based on an entire width of the water-absorbent sheet. The width is preferably 0.15 times or more, from the viewpoint of obtaining a region for diffusing a liquid and preventing a liquid leakage from a longitudinal direction of the water-absorbent sheet, and the width is preferably 0.45 times or less, from the viewpoint of extending diffusion of a liquid in a longitudinal direction and obtaining a permeation rate. When the width of the central region W1 becomes too large, a distance of a liquid transport pathway in a width direction of a water-absorbent sheet **1** becomes long, so that diffusion in a longitudinal direction is likely to be worsened, and that a permeation rate is also likely to be delayed.

The water-absorbent sheet **1** in the present invention is provided with linear embossing **4** in a direction from each of tips **3** of wavy forms formed by the wavy embossing **2,** preferably each of tops **3** of the wavy forms, towards an edge portion contouring along the longitudinal direction of the water-absorbent sheet **1,** thereby improving diffusion in a width direction of the water-absorbent sheet **1,** and intersecting with a flow of a liquid moving in a longitudinal direction, thereby suppressing a liquid leakage in a longitudinal direction. In the present invention, certain effects are exhibited by providing linear embossing **4** from each of tips **3** of wavy forms. It is preferable that linear embossing **4** from each of tops **3** of wavy forms is provided, from the viewpoint of minimizing the influence in the closed region mentioned above and suppressing the lowering of a permeation rate. Here, the linear embossing is not necessarily provided in all the tips, and plural lines of linear embossing may be provided from one tip. In a case where wavy embossing is a single line, it is more preferable that a single line of linear embossing is provided from one of tips. The shape of the linear embossing in the present specification is not particularly limited, and it may be linear or curved. In the present specification, the tops of the wavy forms refer to parts of embossing including tips of wavy forms and vicinity thereof, for example, a part of embossing spreading in left and right centering about tops of wavy forms, the embossing part being within 0.3 times the wavelength of the wavy embossing.

One of the features of the present invention resides in that a branched structure of embossing formed by the wavy embossing **2** and the linear embossing **4** has an approximate Y-shaped form. In the tips of the wavy embossing, since embossing is overlapping in the branched structure as described above, the lowering of water absorption properties of a water-absorbent sheet caused by a closed region formed in the surrounding of emboss overlapping parts can be avoided. As described above, since the embossing shape of the wavy emboss and the embossing shape of the linear emboss may be linear or curved, the embossing constituting a branched structure in the above approximate Y-shaped form may be a linear embossing or curved embossing, or may be a mixture of the both.

In a case where the wavy embossing is plural lines, the above-mentioned linear embossing is provided only on both ends of the existing wavy embossing of the plural lines, in other words outermost two lines of wavy embossing in a width direction of a water-absorbent sheet. In a case where linear embossing is provided on wavy embossing other than both the ends, water absorption properties are likely to be lowered by forming a spatially closed region with embossing overlapping parts between the wavy embossing.

The water-absorbent sheet **1** in the present invention is provided with non-embossing regions (W2, W2') that is not provided with embossing, extending along the longitudinal direction in the edge portion contouring along the longitudinal direction of the sheet, and in this region diffusion of a body liquid is lowered, thereby suppressing a liquid leakage in a width direction of a water-absorbent sheet.

Each of the non-embossing regions (W2, W2') shown in Figure 1 in the present invention has a width of preferably within the range of from 0.05 to 0.20 times, and more preferably within the range of from 0.08 to 0.15 times, that of the entire width of the water-absorbent sheet **1.** The width is preferably 0.05 times or more, from the viewpoint of suppressing a liquid leakage from a water-absorbent sheet from a width direction, and the width is preferably 0.20 times or less, from the viewpoint of suppressing the lowering of a permeation rate as a water-absorbent sheet caused by undesirably having exceedingly enlarged region for lowering diffusion.

The embossing provided on the water-absorbent sheet in the present invention has an areal percentage within the range of from 3 to 25%, preferably from 4 to 20%, and more preferably from 5 to 15%, of the entire area of the water-absorbent sheet. The areal percentage is preferably 3% or more, from the viewpoint of obtaining a distance for a liquid transport pathway of the embossing part, thereby extending the liquid diffusion and speeding up a permeation rate, and the areal percentage is preferably 25% or less, from the viewpoint of preventing the liquid from being diffused which could be possibly take place before allowing it to be absorbed to the water-absorbent sheet, preventing a liquid leakage from a water-absorbent sheet, and softening a feel of the water-absorbent sheet. In the present specification, the entire area of the water-absorbent sheet refers to an entire area of one side of the water-absorbent sheet in a case where one side of the water-absorbent sheet is provided with embossing, or an entire area of both the sides in a case where both the sides of the water-absorbent sheet are provided with embossing.

A method of providing embossing on a water-absorbent sheet in the present invention includes a method using a pressure, heat or an adhesive, and a method of a combination thereof may be also used.

In addition, the water-absorbent sheet of the present invention may properly be formulated with an additive such as a deodorant, an antibacterial agent, or a gel stabilizer.

An absorbent article can be obtained using the water-absorbent sheet of the present invention. Specifically, an absorbent article of the present is obtained by sandwiching a water-absorbent sheet of the present invention between a liquid-permeable sheet and a liquid-impermeable sheet. As the liquid-permeable sheet and the liquid-impermeable sheet, known sheets used in the field of the present invention can be used, and as to a method of sandwiching with these sheets, known methods can be employed.

EXAMPLES

The present invention will be specifically described hereinbelow by the Examples, without intending to limit the scope of the present invention thereto.

The properties of the water-absorbent sheet were measured in accordance with the following methods.

< Evaluations of Total Permeation Rate and Amount of Re-wet of Water-Absorbent Sheet and Diffusion Length of Liquid >

A rectangular strip of a water-absorbent sheet of 10 × 30 cm, cut in a manner that a longitudinal direction thereof is to be in a length direction (machine feeding direction) of a hydrophilic nonwoven fabric, was furnished and used as a sample.

In a 10 L vessel were placed 60 g of sodium chloride, 1.8 g of calcium chloride dihydrate, 3.6 g of magnesium chloride hexahydrate, and a proper amount of distilled water to completely dissolve. Next, 15 g of an aqueous 1% by mass poly(oxyethylene) isooctylphenyl ether solution was added thereto, and distilled water was further added to adjust the weight of the overall aqueous solution to 6000 g. Thereafter, the mixed solution was colored with a small amount of Blue No.1 to prepare a test solution.

On an upper part of a sample (water-absorbent sheet) was placed a polyethylene air-through style porous liquid-permeable sheet having the same size as the sample (10 × 30 cm) and a basis weight of 22 g/m$^2$. In addition, underneath the sample was placed a polyethylene liquid-impermeable sheet having the same size and basis weight as the sheet, to prepare a simple absorbent article. A cylindrical cylinder having an inner diameter of 3 cm was placed near the central section of this absorbent article, and a 50 mL test solution was supplied into the cylinder at one time. At the same time, a time period until the test solution completely disappeared from the internal of the cylinder was measured with a stopwatch, which was referred to as a first permeation rate (sec). Next, the same procedures were carried out 30 minutes thereafter and 60 minutes thereafter, to measure second and third permeation rates (sec). A total of the number of seconds for the first to third permeation rates was referred to as a total permeation rate. In addition, after the termination of the measurements at each of the permeation rates, the presence or absence of a liquid leakage in a width direction of a water-absorbent sheet was visually confirmed.

After 120 minutes from the start of the feed of the first test liquid, the cylinder was removed, filter papers (about 80 sheets) of 10 cm each side, of which mass was previously measured (Wa (g), about 70 g), were stacked near the liquid feeding position of the absorbent article, and a 5 kg weight having a size of 10 cm × 10 cm was placed thereon. After 5 minutes of applying a load, the mass (Wb (g)) of the filter papers was measured, and an increased mass was defined as the amount of re-wet (g) as follows.

$$\text{Amount of Re-wet (g)} = Wb - Wa$$

After the termination of the measurements of amounts of re-wet, a spread of a test solution in a longitudinal direction of a water-absorbent sheet, a sample, was measured as a diffusion length (cm).

< Slope Leakage Test >

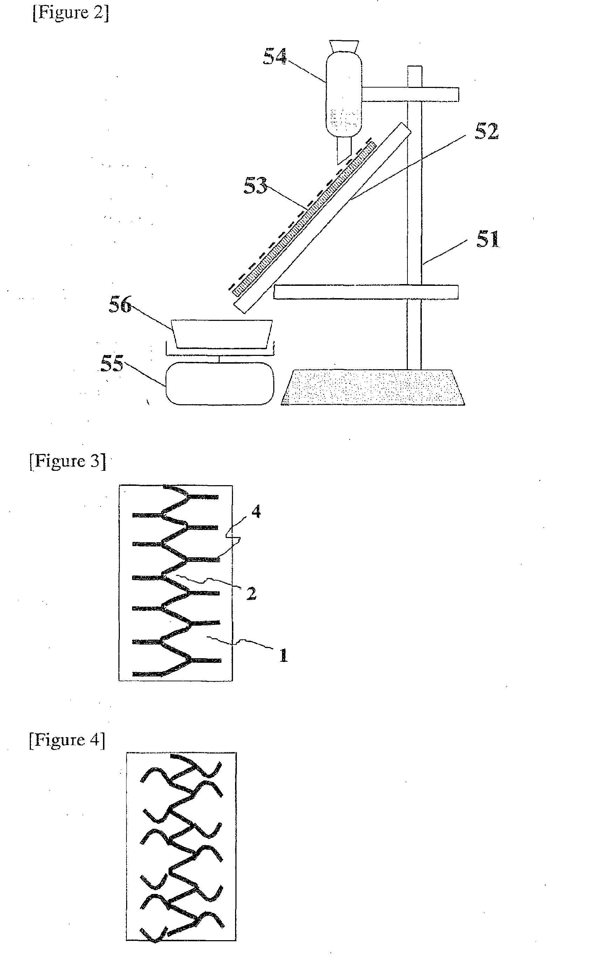

A slope leakage test was conducted using an apparatus shown in Figure 2.
Schematically, a mechanism is as follows. A commercially available stand **51** for experimental facilities was used to slope an acrylic plate **52** and fixed, the above-mentioned test solution was then supplied to a water-absorbent sheet **53** placed on the acrylic plate from a dropping funnel **54** positioned vertically above the sheet, and an amount of a test solution detained on a metallic tray **56** placed in a lower part of an acrylic plate **52** was measured as an amount of leakage with a balance **55.** The detailed specifications are given hereinbelow.
An acrylic plate **52** has a length in the direction of the slope plane of 45 cm, and fixed so that an angle formed with a stand **51** against the horizontal is $45° \pm 2°$. The acrylic plate **52** had a width of about 100 cm and a thickness of about 1 cm, and plural water-absorbent sheets **53** could be concurrently measured. The acrylic plate **52** had a smooth surface, so that a liquid was not detained or absorbed to the plate.
A dropping funnel **54** was fixed at a position vertically above the sloped acrylic plate **52** using a stand **51**. The dropping funnel **54** had a volume of 100 mL, and an inner diameter of a tip end portion of about 4 mm$\phi$, and an aperture of the cock was adjusted so that a liquid was supplied at a rate of 8 mL/seconds.
A balance **55** on which a metallic tray **56** was placed was set at a lower part of the acrylic plate **52,** and all the test solutions flowing down the acrylic plate was received as leakage, and its mass was recorded to the accuracy of 0.1 g.
A slope leakage test using an apparatus as described above was carried out in accordance with the following procedures. A rectangular strip of a water-absorbent sheet **53** of $10 \times 30$ cm, cut in a manner that a longitudinal direction thereof is to be in a length direction (machine feeding direction) of the hydrophilic nonwoven fabric, was used as a sample, and the mass thereof was measured. Next, an air through-style polyethylene liquid-permeable nonwoven fabric (basis weight: 22 g/m$^2$) of the same size was attached from an upper part thereof, and further a polyethylene liquid-impermeable sheet having the same basis weight of the same size was attached from a lower part thereof to prepare a simple absorbent article. The simple absorbent article was adhered on the acrylic plate **52** (in order not to stop leakage intentionally, the bottom end of the water-absorbent sheet **53** was not adhered to the acrylic plate **52**).
Marking was put on the water-absorbent sheet **53** at a position 2 cm away in a downward direction from a top end thereof, and a supplying inlet for the dropping funnel **54** was fixed so that the inlet was positioned at a distance 8 mm $\pm$ 2 mm vertically above the marking.
A balance **55** was turned on, and the indication was tared so that the indication was zero, and thereafter 80 mL of the above-mentioned test solution was supplied at one time to the dropping funnel **54.** An amount of liquid poured into a metallic tray **56** after the test solution was allowed to flow over a sloped acrylic plate **52** without being absorbed into a water-absorbent sheet **53** was measured, and this amount of liquid is referred to a first leakage amount (mL). The numerical value for this first leakage amount (mL) was denoted as LW1.
Second and third test solutions were supplied in 10-minute intervals from the beginning of the first supply, and second and third leakage amounts (mL) were measured, and the numerical values therefor were respectively denoted as LW2 and LW3.
Next, a leakage index was calculated in accordance with the following equation. The closer the index approaches to zero, the smaller the leakage amount at a slope of a water-absorbent sheet, especially an initial leakage amount, whereby it is judged to be an excellent water-absorbent sheet.

$$\text{Leakage Index:} \quad L = LW1 \times 10 + LW2 \times 5 + LW3$$

< Measurement of Thickness of Water-Absorbent Sheet >

The thickness of the resulting water-absorbent sheet was measured using a thickness measurement instrument (manufactured by OZAKI MFG. Co., LTD., model number: J-B). As the measurement sites, three sites were arbitrarily determined in a lengthwise direction, on the left end, the center, and the right end; for example, in a water-absorbent sheet having sizes of 10 cm $\times$ 30 cm, the left end was set at a site 3 cm away from the left side, the center was set at a site 15 cm away therefrom, and the right end was set at a site 27 cm away therefrom. As the width direction, a uniform central part was measured.
The measurement value for thickness was obtained by measuring three times at each site and averaging the values for each site. Further, the values at the left end, the center, and the right end were averaged, to give a thickness of an overall water-absorbent sheet.

< Overall Evaluation >

Based on the analytical results mentioned above, an overall evaluation was made as a water-absorbent sheet.

S:    Highly excellent as a water-absorbent sheet.
A:    Excellent as a water-absorbent sheet.
B:    Having some problems as a water-absorbent sheet in actual use.
C:    Having poor properties as A water-absorbent sheet that cannot be actually used.

[Example 1]

A spun lace nonwoven fabric (basis weight: 50 g/m$^2$, thickness: 400 $\mu$m, rayon content: 70%, polyethylene terephthalate content: 30%) having a width of 30 cm was spread over a hot melt applicator (manufactured by HALLYS Corporation, Marshall 150) of which heating temperature was set at 150°C, and thereafter a styrene-butadiene-styrene copolymer (softening point: 85°C) was coated on the nonwoven fabric at a basis weight of 20 g/m$^2$ as an adhesive.
Next, a roller spreader (manufactured by HASHIMA CO., LTD., SINTERACE M/C) was charged at its supplying inlet with a sodium polyacrylate crosslinked product (manufactured by Sumitomo Seika Co., Ltd., AQUAKEEP SA55SX-II, median particle size: 360 $\mu$m; water-absorption rate of saline solution: 42 seconds, water retention capacity of saline solution: 35 g/g) as a water-absorbent resin. On the other hand, the adhesive-coated nonwoven fabric mentioned above was spread over a conveyor at the bottom part of the spreader. Next, the spreading roller and the bottom part conveyor were operated, thereby allowing the above-mentioned sodium polyacrylate crosslinked product to evenly overlay over the above-mentioned nonwoven fabric at a basis weight of 270 g/m$^2$.
The overlaid product obtained was pressed from a top part with a spun lace nonwoven fabric coated with a styrene-butadiene-styrene copolymer (softening point: 85°C) at a basis weight of 20 g/m$^2$ in the same manner as above, and heat-fused with a laminating machine (manufactured by HASHIMA CO., LTD., straight linear fusing press HP-600LF) of which heating temperature was set at 100°C to integrate, to give an intermediate product A of a water-absorbent sheet.
In the same manner as above, a spun lace nonwoven fabric (basis weight: 50 g/m$^2$, thickness: 400 $\mu$m, rayon content: 70%, polyethylene terephthalate content: 30%) having a width of 30 cm was spread over the hot melt applicator of which heating temperature was set at 150°C, and thereafter a styrene-butadiene-styrene copolymer (softening point: 85°C) was coated on the nonwoven fabric at a basis weight of 6 g/m$^2$ as an adhesive.
Next, the roller spreader was charged at its supplying inlet with a sodium polyacrylate crosslinked product (manufactured by Sumitomo Seika Co., Ltd., AQUAKEEP 10SH-PB, median particle size: 320 $\mu$m; water-absorption rate of saline solution: 3 seconds, water retention capacity of saline solution: 42 g/g) as a water-absorbent resin. On the other hand, the adhesive-coated nonwoven fabric mentioned above was spread over a conveyor at the bottom part of the spreader. Next, the spreading roller and the bottom part conveyor were operated, thereby allowing the above-mentioned sodium polyacrylate crosslinked product to evenly overlay over the above-mentioned nonwoven fabric at a basis weight of 68 g/m$^2$.
The overlaid product obtained was pressed from a top part with a spun lace nonwoven fabric coated with a styrene-butadiene-styrene copolymer (softening point: 85°C) at a basis weight of 6 g/m$^2$ in the same manner as above, and heat-fused with a laminating machine (manufactured by HASHIMA CO., LTD., straight linear fusing press HP-600LF) of which heating temperature was set at 100°C to integrate, to give an intermediate product B of a water-absorbent sheet.
Next, a styrene-butadiene-styrene copolymer (softening point: 85°C) was coated on an upper part of a spun lace nonwoven fabric of the intermediate product B of a water-absorbent sheet in the same manner as mentioned above at a basis weight of 4 g/m$^2$, and thereafter the intermediate product A of a water-absorbent sheet was overlaid on an upper part thereof. Next, the above-mentioned water-absorbent sheets A and B were heat-fused with a laminating machine of which heating temperature was set at 40°C to integrate, to give a laminate sheet.
The resulting laminate sheet was cut into a size of 30 cm in a longitudinal direction and 10 cm in a width direction, and thereafter wires were worked to be arranged on the laminate sheet, so as to form an embossing shape having the properties as shown in Table 1 and shown in Figure 3. The linear embossing **4** is provided in a linear form from each of tips (specifically each apex) of the wavy embossing **2.** Next, these wires were pressed with a hydraulic pressing machine (manufactured by FUTANAKOUKI Co., LTD.) of which heating temperature was set at 100°C for 1 second (pressure against the wires: 38 MPa), thereby embossing work is provided to the laminate sheet, to give a water-absorbent sheet **1.** Next, various kinds of properties of the water-absorbent sheets were measured, the results are shown in Table 2.
[Table 1]

Table 1

| | Embossing Line Width [mm] | Central Region | | Non-Embossing Region | | | | Areal Percentage of Embossing [%] | Patterns of Figures |
|---|---|---|---|---|---|---|---|---|---|
| | | W1 | | W2 | | W2' | | | |
| | | [cm] | Folds Based on Sheet Width | [cm] | Folds Based on Sheet Width | [cm] | Folds Based on Sheet Width | | |
| Ex. 1 | 3 | 2.5 | 0.25 | 1.25 | 0.125 | 1.25 | 0.125 | 7 | FIG. 3 |
| 2 | 3 | 2.5 | 0.25 | 1.25 | 0.125 | 1.25 | 0.125 | 9 | FIG. 4 |
| 3 | 3 | 3.5 | 0.35 | 0.75 | 0.075 | 0.75 | 0.075 | 13 | FIG. 5 |
| 4 | 3 | 1.0 | 0.10 | 2.00 | 0.20 | 2.00 | 0.20 | 7 | FIG. 6 |
| 5 | 3 | 5.0 | 0.50 | 0.50 | 0.05 | 0.50 | 0.05 | 9 | FIG. 7 |
| 6 | 3 | 2.5 | 0.25 | 0.20 | 0.02 | 0.20 | 0.02 | 8 | FIG. 8 |
| 7 | 3 | 1.0 | 0.10 | 4.00 | 0.40 | 4.00 | 0.40 | 4 | FIG. 9 |
| Comp. Ex. 1 | - | - | - | - | - | - | - | - | - |
| 2 | 1 | - | - | 0.00 | 0.00 | 0.00 | 0.00 | 10 | FIG. 10 |
| 3 | 3 | 3.5 | 0.35 | 3.25 | 0.325 | 3.25 | 0.325 | 8 | FIG. 11 |
| 4 | 3 | - | - | 1.50 | 0.15 | 1.50 | 0.15 | 8 | FIG. 12 |
| 5 | 2 | 2.5 | 0.25 | 1.25 | 0.125 | 1.25 | 0.125 | 8 | FIG. 13 |
| 6 | 1 | 2.5 | 0.25 | 1.25 | 0.125 | 1.25 | 0.125 | 2 | FIG. 14 |
| 7 | 3 | 4.5 | 0.45 | 1.75 | 0.175 | 1.75 | 0.175 | 31 | FIG. 15 |

[Comparative Example 1]

A water-absorbent sheet was obtained in the same manner as in Example 1 except that a laminate sheet was not provided with embossing work, and thereafter various kinds of properties were measured. The results are shown in Table 2.

[Examples 2 to 7 and Comparative Examples 2 to 7]

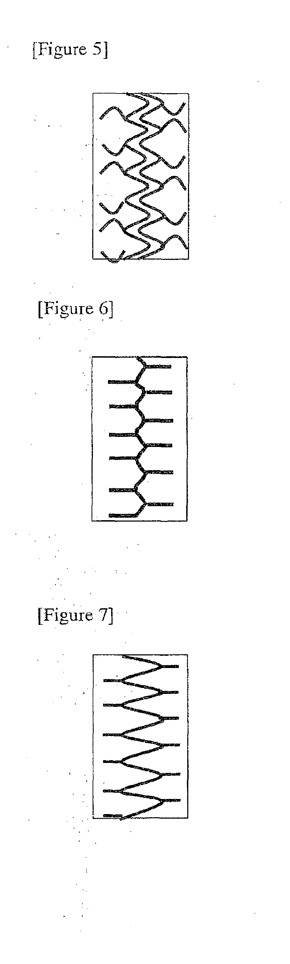

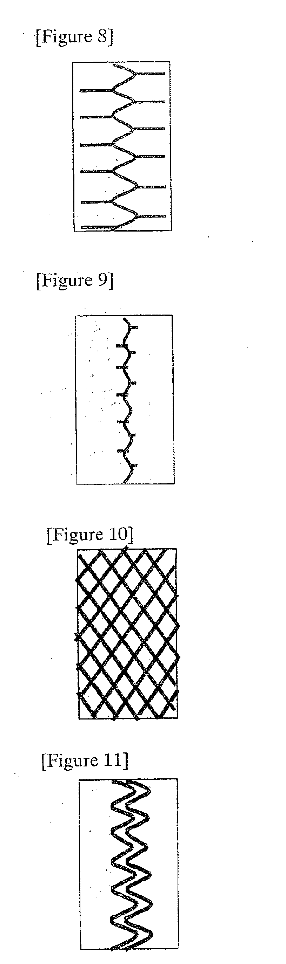

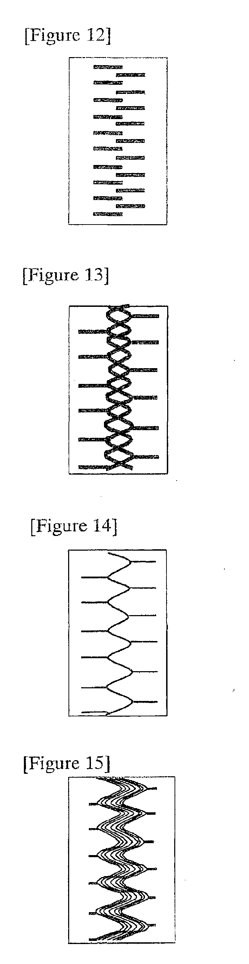

A water-absorbent sheet was obtained in the same manner as in Example 1 except that each of the sheets had the properties as shown in Table 1, and was provided with embossing shapes as shown in Figures 4 to 15, and thereafter various kinds of properties were measured. The results are shown in Table 2.

[Reference Example]

The amount 7.5 g of a sodium polyacrylate crosslinked product (manufactured by Sumitomo Seika Co., Ltd., AQUAKEEP SA55SX-II, median particle size: 360 $\mu$m; water-absorption rate of saline solution: 42 seconds, water retention capacity of saline solution: 35 g/g) as a water-absorbent resin, and 7.5 g of pulverized pulp were dry-blended. The resulting mixture was sprayed to tissue paper having a size of 10 cm $\times$ 30 cm and a weight of 0.75 g, and thereafter tissue paper having the same size and the weight as above was layered over the top, and formed into a sheet. A 196 kPa load was applied to the entire sheet for 30 seconds to press the sheet, thereby giving an absorbent material. Each of the properties for this absorbent material were measured. The results are shown in Table 2.

[Table 2]

Table 2

| | Thickness [mm] | Permeation Rate [sec] | | | | Amount of Rewet [g] | Diffusion Length [cm] | Liquid Leakage in Width Direction | Slope Leakage Test | | | | Overall Evaluation |
|---|---|---|---|---|---|---|---|---|---|---|---|---|---|
| | | 1 | 2 | 3 | Total | | | | LW1 | LW2 | LW3 | Index | |
| Ex. 1 | 2.2 | 19 | 10 | 9 | 38 | 1.7 | 19 | Absence | 0.0 | 1.1 | 3.3 | 9 | S |
| 2 | 2.3 | 22 | 9 | 7 | 38 | 0.7 | 19 | Absence | 0.0 | 0.0 | 1.8 | 2 | S |
| 3 | 2.3 | 17 | 9 | 6 | 32 | 0.4 | 25 | Absence | 0.0 | 5.2 | 20.2 | 46 | A |
| 4 | 2.2 | 18 | 9 | 8 | 35 | 0.7 | 19 | Absence | 1.2 | 1.2 | 3.1 | 21 | A |
| 5 | 2.2 | 25 | 12 | 10 | 47 | 0.5 | 16 | Absence | 0.0 | 0.0 | 0.3 | 0 | A |
| 6 | 2.2 | 20 | 10 | 9 | 39 | 1.1 | 18 | Presence | 0.0 | 0.8 | 2.9 | 7 | A |
| 7 | 2.1 | 21 | 10 | 8. | 39 | 0.3 | 19 | Absence | 5.1 | 3.2 | 0.0 | 67 | A |
| Comp. Ex. 1 | 2.4 | 41 | 27 | 26 | 94 | 2.0 | 15 | Absence | 0.0 | 0.0 | 0.0 | 0 | C |
| 2 | 2.3 | 23 | 11 | 7 | 41 | 0.3 | 24 | Presence | 0.5 | 18.0 | 42.0 | 137 | C |
| 3 | 2.2 | 23 | 11 | 9 | 43 | 0.5 | 21 | Absence | 7.2 | 3.3 | 1.8 | 90 | B |
| 4 | 2.2 | 31 | 17 | 14 | 62 | 0.8 | 18 | Absence | 0.0 | 0.0 | 0.0 | 0 | B |
| 5 | 2.2 | 35 | 23 | 20 | 78 | 1.2 | 17 | Absence | 2.2 | 1.3 | 0.0 | 29 | C |
| 6 | 2.2 | 27 | 11 | 13 | 51 | 0.6 | 17 | Absence | 0.0 | 0.0 | 0.5 | 1 | B |
| 7 | 2.1 | 26 | 11 | 8 | 45 | 0.7 | 24 | Absence | 5.3 | 22.9 | 30.0 | 198 | C |
| Ref Ex. | 3.5 | 19 | 14 | 15 | 48 | 15.1 | 17 | Absence | 0.0 | 0.0 | 0.0 | 0 | - |

From the results shown in Table 2, the water-absorbent sheet of each example having a fast permeation rate, a small amount of re-wet, and favorable slope leakage was obtained. As to the water-absorbent sheets as in Examples 1 and 2 where wavy embossing was a single line, and provided with linear embossing in a straight line or curved line, one each from each tips (top), even higher evaluations were obtained.

On the other hand, in Comparative Examples, all of a sheet not provided with embossment (Comparative Example 1), a sheet of which embossing shape is non-wavy (Comparative Example 2, Comparative Example 4), a sheet not provided with linear embossment (Comparative Example 3), and a sheet of which wavy forms overlap with each other (Comparative Example 5), a sheet of which embossing shape is similar to that of Example 1 but narrower in width of an embossing line, thereby having an areal percentage for embossment lower than a given range (Comparative Example 6), and a sheet of which areal percentage for embossment exceeding a given range (Comparative Example 7) could not simultaneously satisfy both the problems of improvement in permeation rates and prevention of leakage, thereby making it unsatisfactory as a water-absorbent sheet. Further, as can be seen from Reference Example, while leakage could be suppressed because pulp is used in an absorbent material, the amount of re-wet was large and the values for the thickness were high.

[Industrial Applicability]

The water-absorbent sheet of the present invention has a fast permeation rate of a liquid and does not cause a liquid leakage, and can be suitably used in absorbent articles such as disposable diapers.

[Explanation of Numerical Symbols]

1    water-absorbent sheet
2    wavy embossing
3    tip
4    linear embossing
51   stand
52   acrylic plate
53   water-absorbent sheet
54   dropping funnel
55   balance
56   metallic tray
W1  central region
W2  non-embossing region
W2'  non-embossing region

**Claims**

1. A water-absorbent sheet comprising a structure comprising at least an absorbent layer containing a water-absorbent resin, sandwiched between two or more sheets of a hydrophilic nonwoven fabric, wherein a central region **(W1)** along a longitudinal direction of a water-absorbent sheet **(1)** is subjected to:

   one or more continuous wavy embossing **(2)** that is non-overlapping with each other, extending along the longitudinal direction, and linear embossing **(4)** in a direction from each of tips **(3)** of wavy forms formed by the wavy embossing **(2)** towards an edge portion contouring along the longitudinal direction of the water-absorbent sheet **(1),** and

   wherein a branched structure formed by the wavy embossing **(2)** and the linear embossing **(4)** has an approximate Y-shape-form, and
   wherein the edge portion contouring along the longitudinal direction of the water-absorbent sheet **(1)** comprises non-embossing regions **(W2, W2')** not provided with embossing extending along the longitudinal direction, and further wherein an areal percentage of embossment provided on the water-absorbent sheet **(1)** is from 3 to 25% of an entire area of the water-absorbent sheet **(1).**

2. The water-absorbent sheet according to claim 1, wherein the central region **(W1)** has a width within the range of from 0.15 to 0.45 times that of an entire width of the water-absorbent sheet **(1).**

3. The water-absorbent sheet according to claim 1 or 2, wherein each of the non-embossing regions **(W2, W2')** has a width within the range of from 0.05 to 0.20 times the entire width of the water-absorbent sheet **(1).**

4. An absorbent article comprising the water-absorbent sheet as defined in any one of claims 1 to 3, sandwiched between a liquid-permeable sheet and a liquid-impermeable sheet.

**Patentansprüche**

1. Wasserabsorbierende Bahn, die eine Struktur umfasst, die mindestens eine absorbierende Schicht umfasst, die ein wasserabsorbierendes Harz enthält und sandwichartig zwischen zwei oder mehr Bahnen eines hydrophilen Vliesmaterials eingebracht ist, worin eine zentrale Region (W1) entlang der Längsrichtung der wasserabsorbierenden Bahn (1) folgendem unterzogen wird:

   einer oder mehreren kontinuierlichen wellenförmigen Prägungen (2), die einander nicht überlappen und sich entlang der Längsrichtung erstrecken, und

   einer linearen Prägung (4) in einer Richtung von jeder der Spitzen (3) der durch die wellenförmige Prägung (2) gebildeten Wellenformen zu einem Kantenabschnitt, der eine Kontur entlang der Längsrichtung der wasserabsorbierenden Bahn (1) bildet, und worin eine verzweigte Struktur, die durch die wellenförmige Prägung (2) und die lineare Prägung (4) gebildet wird, eine näherungsweise Y-förmige Form aufweist, und worin der Kantenabschnitt, der entlang der Längsrichtung der wasserabsorbierenden Bahn (1) die Kontur bildet, Regionen ohne Prägung (W2, W2') aufweist, die nicht mit einer Prägung versehen sind und sich entlang der Längsrichtung erstrecken, und worin weiterhin ein Flächenprozentwert der Prägung, die auf der wasserabsorbierenden Bahn (1) bereitgestellt ist, von 3 bis 25 % einer Gesamtfläche der wasserabsorbierenden Bahn (1) ausmacht.

2. Wasserabsorbierende Bahn gemäß Anspruch 1, worin die Zentralregion (W1) eine Breite im Bereich des 0,15 bis 0,45-fachen der Gesamtbreite der wasserabsorbierenden Bahn (1) aufweist.

3. Wasserabsorbierende Bahn gemäß Anspruch 1 oder 2, worin jede der Regionen ohne Prägung (W2, W2') eine Breite innerhalb des Bereichs des 0,05 bis 0,20-fachen der gesamten Breite der wasserabsorbierenden Bahn (1) aufweist.

4. Absorbierendes Erzeugnis, das die wasserabsorbierende Bahn gemäß irgendeinem der Ansprüche 1 bis 3 sandwichartig zwischen eine Flüssigkeits-durchlässige Bahn und eine Flüssigkeits-undurchlässige Bahn eingebracht umfasst.

**Revendications**

1. Feuille absorbant de l'eau comprenant une structure comprenant au moins une couche absorbante contenant une résine absorbant de l'eau, prise en sandwich entre deux ou plus de feuilles d'un tissu hydrophile non tissé, dans laquelle une région centrale (W1) le long d'une direction longitudinale d'une feuille absorbant de l'eau (1) est soumise à :

   un ou plusieurs gaufrage(s) onduleux continu(s) (2) qui ne se chevauchent pas, s'étendant le long de la direction longitudinale, et du gaufrage linéaire (4) dans une direction à partir de chacune de pointes (3) de formes onduleuses formées par le gaufrage onduleux (2) vers une portion de bord contournant le long de la direction longitudinale de la feuille absorbant de l'eau (1), et dans laquelle une structure ramifiée formée par le gaufrage onduleux (2) et le gaufrage linéaire (4) a une forme approximative de Y, et dans laquelle la portion de bord contournant le long de la direction longitudinale de la feuille absorbant de l'eau (1) comprend des régions de non-gaufrage (W2, W2') non pourvues de gaufrage s'étendant le long de la direction longitudinale, et en outre

dans laquelle un pourcentage de zone de gaufrage réalisé sur la feuille absorbant de l'eau (1) est de 3 à 25 % d'une zone entière de la feuille absorbant de l'eau (1).

2. Feuille absorbant de l'eau selon la revendication 1, dans laquelle la région centrale (W1) a une largeur à l'intérieur de la plage allant de 0,15 à 0,45 fois celle d'une largeur entière de la feuille absorbant de l'eau (1).

3. Feuille absorbant de l'eau selon la revendication 1 ou 2, dans laquelle chacune des régions de non-gaufrage (W2, W2') a une largeur à l'intérieur de la plage allant de 0,05 à 0,20 fois la largeur entière de la feuille absorbant de l'eau (1).

4. Article absorbant comprenant la feuille absorbant de l'eau telle que définie dans l'une quelconque des revendications 1 à 3, prise en sandwich entre une feuille perméable à du liquide et une feuille imperméable à du liquide.

[Figure 1]

[Figure 2]

[Figure 3]

[Figure 4]

[Figure 5]

[Figure 6]

[Figure 7]

[Figure 8]

[Figure 9]

[Figure 10]

[Figure 11]

[Figure 12]

[Figure 13]

[Figure 14]

[Figure 15]

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP HEI9510889 B
- JP HEI857311 B
- JP HEI10272155 B